# EUROPEAN PATENT SPECIFICATION

(11) **EP 1 251 844 B1**
(45) Date of publication and mention of the grant of the patent: **03.12.2008**
(21) Application number: 00968147.9
(22) Date of filing: 18.10.2000
(51) Int. Cl.: A61K 31/198, A61P 1/10

(54) **LAXATIVE PREPARATION CONTAINING L-ARGININE**
ABFÜHRZUSAMMENSETZUNG,WELCHE L-ARGININE ENTHÄLT
PREPARATION LAXATIVE CONTENANT DE LA L-ARGININE

(30) Priority: 18.10.1999 GB 9924636
(43) Date of publication of application: 30.10.2002
(73) Proprietor: Norgine BV, 1101 CA Amsterdam Zuidoost (NL); President and Fellows of Harvard College, Cambridge, MA 02138-5701 (US)
(72) Inventor: BARRAS, Norman Norgine Research Limited, Northwood, Middlesex HA6 2RN (GB); HECHTMAN, Herbert, B., Chestnut Hill, MA 02467 (US)
(74) Representative: Silveston, Judith
(86) International application number: PCT/GB2000/004017
(87) International publication number: WO 2001/028538

(56) References cited:
- US-A- 5 693 312
- KARAN, R. S. ET AL: "Effect of acute and chronic administration of L-arginine on morphine-induced inhibition of gastrointestinal motility" INDIAN J. PHYSIOL. PHARMACOL. (2000), 44(3), 345-349 , XP001012477
- STORR M. ET AL: "[Motility-modifying drugs]. MOTILITATSMODIFIZIERENDE PHARMAKA." INTERNIST, (2000) 41/12 (1318-1330). , XP001012467
- CALIGNANO, A. ET AL: "Endogenous nitric oxide modulates morphine-induced constipation" BIOCHEM. BIOPHYS. RES. COMMUN. (1991), 181(2), 889-93 , XP001010273
- IZZO, ANGELO A. ET AL: "NG-Nitro-L-arginine methyl ester modulates intestinal secretion and motility produced by carbachol" EUR. J. PHARMACOL. (1994), 271(1), 31-5 , XP001012470
- MASCOLO, NICOLA ET AL: "Nitric oxide and castor oil-induced diarrhea" J. PHARMACOL. EXP. THER. (1994), 268(1), 291-5 , XP001012471

## Description

The present invention relates generally to laxatives.

Constipation is a widespread condition which generally gives rise to discomfort. The physical presence of faeces retained in the colon and/or the rectum gives rise to a feeling of malaise and headaches. In extreme cases of prolonged constipation dyschezia may result from the presence of scybala or faecaliths in the rectum. Dyschezia may also result from the inhibition of the defecation reflex in response to pain arising from haemorrhoids, ulceration of the anal mucosa or wounds (surgical or traumatic) of the anus.

Laxatives are agents that promote and assist defecation. There are four general types of laxatives that are currently available: 1) bulk-forming; 2) osmotic; 3) stimulatory; 4) softening agents. The present application is directed towards a new class of laxative.

With bulk laxatives, an increase in the volume of the contents of the colon stimulates local peristaltic activity and increases the strength of stimulation of the central reflexes concerned with defecation. Bulk laxatives simulate the effect of foods containing indigestible and non-absorbed constituents.

In contrast to bulk-forming laxatives, osmotic laxatives act to retain water in the colonic lumen thereby counteracting the normal dehydrating action of the colon. By suppressing the dehydration action of the colon, the osmotic laxative produces a faecal stream which is softer, bulkier and easier to expel.

Stimulatory laxatives increase peristalsis by stimulating the mucosa of the gut (probably by initiating local reflexes), the impulses arising in the mucosa and being transmitted through the intramural plexuses to the smooth muscle of the intestine. These agents can sometimes cause abdominal cramps; prolonged use can lead to deterioration of intestinal function, and can result in atonic colon.

Softening agents are generally surface-active compounds which act on the gastrointestinal tract in a manner similar to a detergent and produce softer faeces.

All of the methods so far mentioned vary in their speed of action, have a number of side effects and are not always effective. It is an objective of the present invention to provide a laxative that overcomes or ameliorates some or all of these problems.

Calignano et al. (1991) Biochem. Biophys. Res. Commun., 181(2) reports that L-Arginine reverses the constipation induced by morphine in mice.

Compositions comprising L-arginine are known to be effective in the treatment of haemorrhoids and haemorrhoidal pain (as described in WO96/32081). It has now been surprisingly found that formulations comprising L-arginine are effective as a laxative.

The invention provides laxative treatment of the mammalian, especially human, gastrointestinal (GI) tract using a preparation comprising the amino acid L-arginine, a derivative thereof, or pharmaceutically acceptable salt thereof in a pharmaceutically acceptable carrier substance.

The invention provides a treatment for medical conditions of the gastrointestinal tract, including constipation. As used herein, the term "gastrointestinal tract" is defined as the part of the body which includes the oesophagus, stomach, small and large intestines, and includes the colon, rectum and anus. The terms "topical" and "topical application" are defined as application of an active substance to surfaces of the body, and are used herein to mean application to an area of the gastrointestinal tract. This mode of administration is sometimes also known as "local application. As used herein, the term "effective amount" refers to an amount of L-arginine, a derivative thereof or a pharmaceutically acceptable salt thereof that results in at least partial alleviation or mitigation of the medical condition. The terms "medicament", "composition" and "preparation" are used herein interchangeably.

The present invention provides the use of L-arginine, or a derivative thereof, or a pharmaceutically acceptable salt thereof for the manufacture of a medicament for the treatment of endogenous constipation. The invention also provides the use of L-arginine, a derivative thereof or a pharmaceutically acceptable salt thereof for the manufacture of a medicament for use as a laxative in a patient without constipation who should be prevented from straining at the stool, for example a patient who suffers from a hernia or cardiovascular disease, haemorrhoids or other anorectal disorders. The invention further provides the use of L-arginine, a derivative thereof, or a pharmaceutically acceptable salt thereof for the manufacture of a medicament for clearing the rectum and/or the colon of a mammal, wherein the colon and/or rectum are cleared prior to carrying out a diagnostic, therapeutic or surgical procedure on the colon, rectum or anus or elsewhere in the abdomen. The medicament is especially one that enables topical application of the L-arginine, a derivative thereof, or a pharmaceutically acceptable salt thereof, that is to say, application to an area of the gastrointestinal tract.

In the uses according to the present invention, the recipient of the medicament is especially a human. The L-arginine, derivative thereof, or pharmaceutically acceptable salt thereof is applied, in the form of a medicament, to the gastrointestinal tract, especially to an area thereof. That area is, for example, the gastrointestinal tract beyond the pyloric sphincter, for example, the gastrointestinal tract beyond the duodenum, for example, the gastrointestinal tract beyond the ileum, for example, the colon and beyond, for example the rectum or anal canal. The area of the gastrointestinal tract to which the active substance is applied may be the colon and/or rectum.

The medicament comprising the L-arginine, a derivative thereof, or a pharmaceutically acceptable salt thereof should be such that the active substance is applied to the desired site. The medicament is administered in an amount such that an effective amount of the L-arginine, a derivative thereof, or a pharmaceutically acceptable salt thereof is administered to the desired site. The medicament generally comprises one or more pharmaceutically acceptable carriers.

The present invention is concerned with the treatment of endogenous constipation. Constipation may be acute, chronic or refractory and may be endogenous or exogenous. Exogenous constipation is caused by external agents.

The invention is also concerned with the use of L-arginine, a derivative thereof, or a pharmaceutically acceptable salt thereof as a laxative, for example in promoting and/or assisting defecation. While laxatives are often used by those suffering from constipation, they may also be used to promote and/or assist defecation by subjects who do not have constipation as such but who should be prevented from straining at the stool, for example, patients suffering from hernia or cardiovascular disease. Laxatives may also be useful to patients with haemorrhoids and other anorectal disorders.

Clearing the large intestine, in particular the colon and/or rectum, also known as intestinal lavage, is important for diagnostic, therapeutic and surgical procedures on the colon, rectum or anus. It may also be desired to facilitate such procedures in other regions of the abdomen, for example, before examination, for example, radiological examination of the kidneys or other abdominal or retroperitoneal structures. The term "colon and/or rectum" is used herein. However, it will be appreciated that an agent administered to the colon will generally also reach the rectum. Similarly, clearance of the colon will generally also result in clearance of the rectum. Conversely, application of an agent to the rectum or clearance of the rectum will not necessarily affect the colon.

Suitable pharmaceutically acceptable L-arginine salts for use according to the present invention include, for example, L-arginine monohydrochloride, L-arginine free base, L-arginine L-aspartate salt, L-glutamyl L-arginine, L-arginine hydrochloride, L-arginine hydroxamate, L-arginine phosphate, L-argininic acid, L-arginine benzylidene, L-arginine diflavinate, L-arginine dipicrate and L-arginine picrate. The hydrochloride salt is particularly preferred. Suitable derivatives of L-arginine include, for example, L-arginine methyl ester dihydrochloride, L-arginine agarose, L-arginine ethyl ester dihydrochloride, L-arginine-4-methoxy-β-naphthylamide, L-arginine methyl ester p-toluenesulfonyl derivative, L-arginine-β-naphthylamide hydrochloride, L-arginine-p-nitroanilide dihydrochloride, L-argininamide dihydrochloride, L-arginin-N^{G}-amine (flavionate salt), argininosuccinic acid and L-argininamide. The term "pharmaceutically acceptable salts thereof" includes salts of L-arginine and salts of L-arginine derivatives. Generally the L-arginine derivative is preferably not a surfactant, for example not an acyl arginine surfactant, for example not N-myristoyl-L-arginine methyl ester hydrochloride.

Preferably, L-arginine is used. A particularly suitable salt of L-arginine is L-arginine hydrochloride.

In one embodiment of the invention, the medicament is suitable for topical application via the anal canal, i.e. rectally. The carrier for the topical application can be any inert substance suitable for topical administration in the gastrointestinal tract, in particular for administration rectally to the colon and/or rectum, for example, a water-soluble jelly, for example, hydroxy methyl cellulose or a water-based gel, for example, as available from Johnson and Johnson under the name "K-Y Jelly"; a hydrogel; an anal suppository base or a cream.

In general, a carrier with a density higher than water, for example, a water based gel for example, as available from Johnson and Johnson under the name "K-Y Jelly" or a suppository base, is capable of subjecting an area to a prolonged exposure to the active ingredient.

Hydrogels may be selected that release the active substances at a pre-determined rate.

The amount of topical medicament administered is generally in the range of from 0.01 ml to 10 ml. The concentration of L-arginine in the topical medicament is generally in the range of from 0.1 mg to 800 mg per ml, preferably from 10 mg to 700 mg per ml, more preferably from 50 mg to 500 mg per ml, for example 400 mg per ml. The preferred amounts of L-arginine, or derivative or salt thereof, calculated as L-arginine, are accordingly from 0.1 to 7000 mg, more preferably from 0.5 to 5000 mg, for example, from 4 to 4000 mg.

Upon administration of the L-arginine, derivative thereof or pharmaceutically acceptable salt thereof in a suitable carrier to the rectum, anus or colon, defecation generally results and the symptoms of constipation are reduced. Hence the use of L-arginine, derivatives thereof and salts thereof according to the present invention enables the clearance of faecal material retained in the lower gastrointestinal tract, for example the colon and/or rectum at the time of administration.

The invention may or may not rely on the delivery of the active substance to the rectum, anus or colon. The mechanism of action of the treatment is at present not known. It is possible that the mechanism involves NO release.

At least two types of NO synthase enzymes contribute to production of NO. An inducible NO synthase depends upon protein synthesis of the enzyme before NO production begins. A constitutive NO synthase enzyme is present in endothelial cells, platelets, brain, and smooth muscle cells. It is possible that L-arginine directly activates the constitutively expressed NO synthase enzyme to cause production of NO or a related compound, since defecation generally occurs very soon after application of this agonist. Following NO production, guanylate cyclase is activated in smooth muscle which leads to the formation of cyclic guanosine 3', 5'-monophosphate (cGMP), a transduction mechanism for a number of stimuli in addition to those leading to muscle relaxation and vasodilatation.

In a preferred embodiment of the invention, the medicament is suitable for oral administration. In this case, the medicament may be a tablet, pill, capsule or powder. In a particularly preferred embodiment, the medicament, especially a tablet, is coated with an enteric coating which is selected so that it dissolves at a desired location in the gastrointestinal tract, such as at the terminal ileum. A number of such enteric coatings are known.

Many of these take the form of pH sensitive polymers. The polymer is coated onto the surface of the medicament and the active substance is released only after the coating has dissolved. Enteric coatings that may be used in conjunction with the medicaments of the invention include, for example, cellulose acetate 1,2 benzedicarboxylate, hexadecan-1-ol, cellulose ethyl ether, liquid glucose, cellulose 2-hydroxyethylether, cellulose 2-hydroxypropylether, cellulose 2-hydroxypropyl methyl ether, cellulose hydrogen 1,2 benzene dicarboxylate 2-hydroxypropylmethyl ether, maltodextrin, cellulose methylether, polymethacrylates, shellac, confectioner's sugar, titanium oxide, carnauba wax, microcrystalline wax, zein, gelatin, polyvinyl ethanol and cellulose carboxymethyl ether sodium salt. It is also possible to blend the L-arginine, derivative or salt with a polymer before coating the medicament or to use an appropriate polymer matrix. Microspheres, for example, having controlled release coatings may be used. Using such systems the active ingredient is released at the desired location and sustained or controlled release of the active ingredient at the site can be achieved. Drug delivery systems that are colon specific may also be used in conjunction with the present invention in order to effect release of the active substance in the colon. Drug delivery systems are known that effect selective targeting to the ascending, transverse or descending sections of the colon. For example, coatings are known that are degraded by colonic bacteria. Many further means and formulations for achieving controlled, sustained or targeted release are known.

In summary, a desired sustained, controlled and/or targeted release may be achieved, for example, by use of an enteric coating; by use of a sustained release or controller release formulation, for example, coated microspheres or an appropriate polymer or polymer matrix; or by a formulation or a drug delivery system that is targeted or specific for a particular region of the gastrointestinal tract. Such means may be used singly or in any desired combination. Accordingly, a sustained and/or controlled release medicament may comprise more than one means for achieving the desired effect, for example, a medicament may comprise microspheres or a core comprising a polymer to achieve sustained and/or controlled release with an enteric coating to achieve targeted release.

The amount of the L-arginine, derivative thereof or pharmaceutically acceptable salt thereof administered is preferably such that an effective quantity of the active substance reaches the rectum, anus or colon. In a preferred embodiment, from 0.1 mg to 5 g of L-arginine reach the rectum, anus or colon, more preferably from 10 mg to 1 g reach the rectum, anus or colon, still more preferably 50 mg to 800 mg reach the rectum, anus or colon. Preferably, the requisite amount of the active substance reaches the descending colon or the rectum.

In a further embodiment, the pharmaceutically acceptable carrier is a solution. In a further embodiment, the pharmaceutically acceptable carrier is an enema solution. It is preferably not a solution of L-arginine in saline for oral administration.

The invention further provides the use of L-arginine, a derivative thereof, or a pharmaceutically acceptable salt thereof in conjunction with one or more further laxative medicaments. In a preferred embodiment, a further laxative medicament is a bulk-forming laxative, an osmotic laxative, a stimulatory agent or a softening agent. The further laxative and the L-arginine, a derivative thereof, or a pharmaceutically acceptable salt thereof may be administered simultaneously, separately or sequentially. When used in conjunction with a bulk laxative comprising a cellulose ether, especially a water-soluble, non-ionic cellulose ether, it is preferable that the L-arginine active ingrdient is not an L-arginine derivative that is a surfactant, particularly not an acyl arginine ester that is a surfactant, for example not N-myristoyl-L-arginine methyl ester hydrochloride.

A number of methods of surgery, therapy and diagnosis require good access to the gastrointestinal tract, so clearing the gastrointestinal tract, in particular the lower parts of the gastrointestinal tract, is carried out using, for example, polyethylene glycol. However, effective clearance is difficult to achieve.

As indicated above, the invention includes the use of L-arginine, a derivative thereof or a pharmaceutically acceptable salt thereof to clear the colon and/or rectum by administering a composition comprising the L-arginine, a derivative thereof or a pharmaceutically acceptable salt thereof to the gastrointestinal tract, especially to an area thereof, for example, to the rectum or the colon. The composition comprising L-arginine, a derivative thereof or a pharmaceutically acceptable salt thereof may be used in conjunction with a further active ingredient with a complimentary mode of action, for example a rectal product comprising a softening agent or a bulk forming laxative given orally, as described above. In a preferred embodiment, the pharmaceutically acceptable carrier is a cream, an anal suppository base, a hydrogel, hydroxy methyl cellulose; or a water-soluble jelly for example, a water based gel, for example, as available from Johnson and Johnson under the name "K-Y Jelly". Alternatively, the pharmaceutically acceptable carrier may be an enema solution. In a particularly preferred embodiment, the medicament is in a form suitable for oral ingestion, as described above and also as described below in relation to treatment of constipation.

As indicated above, such a regime may be used for at least partially clearing the colon prior to a diagnostic procedure or prior to surgery carried out on the colon or rectum, for example, for example colonoscopy or sigmoidoscopy, or carried out on other structures elsewhere in the abdomen.

The present invention is concerned with treatment of endogenous constipation. As indicated above, constipation may be acute, chronic or refractory and may be endogenous or exogenous.

According to the present invention endogenous constipation may be treated using L-arginine, a derivative thereof or a salt thereof, administered either topically via the rectum using one of the topical medicaments described above or, preferably, administered orally using a medicament that has, preferably, a sustained, controlled and/or targeted release formulation. As pointed out above, sustained and/or controlled release formulations are known in the art as are means for achieving targeted release. A desired sustained, controlled and/or targeted release may be achieved, for example, by use of an enteric coating; by use of a sustained release or controlled release formulation, for example, coated microspheres or an appropriate polymer or polymer matrix; or by a formulation or a drug delivery system that is targeted or specific for a particular region of the gastrointestinal tract. Such means may be used singly or in any desired combination. Accordingly, a sustained and/or controlled release medicament may comprise more than one means for achieving the desired effect, for example, a medicament may comprise microspheres or a core comprising a polymer to achieve sustained and/or controlled release with an enteric coating to achieve targeted release.

For the treatment of endogenous constipation, the L-arginine, derivative or salt thereof may preferably be released after the pyloric sphincter, as example, as described above. Enteric coatings suitable for release after passage of the medicament through the stomach are known. Release may occur, for example, in the duodenum, ileum or colon. Coatings are known for example, which dissolve in the terminal ileum, releasing active substance in the colon. It may be preferable to have slow controlled release of the active substance as the medicament passes through the gastrointestinal tract, for example, through area thereof, for example as described above. As indicated above, a suitable sustained/controlled release formulation may be coated with an enteric coating, for example to achieve sustained and controlled release along the gastrointestinal tract, for example, starting at the duodenum, or starting at the colon. For release in the colon, a colon-specific drug delivery system may be used, optionally in combination with a sustained/controlled release formulation.

An enteric coating may be selected so that it dissolves at a desired location in the gastrointestinal tract, for example, at the duodenum or terminal ileum. A number of such enteric coatings are known. Many of these take the form of pH sensitive polymers. The polymer is coated onto the surface of the medicament and the active substance is released only after the coating has dissolved. Enteric coatings that may be used in conjunction with the medicaments for the treatment of constipation including opiate-induced constipation include, for example, cellulose acetate 1,2 benzedicarboxylate, hexadecan-1-ol, cellulose ethyl ether, liquid glucose, cellulose 2-hydroxyethylether, cellulose 2-hydroxypropylether, cellulose 2-hydroxypropyl methyl ether, cellulose hydrogen 1,2 benzene dicarboxylate 2-hydroxypropylmethyl ether, maltodextrin, cellulose methylether, polymethacrylates, shellac, confectioner's sugar, titanium oxide, carnauba wax, microcrystalline wax, zein, gelatin, polyvinyl ethanol and cellulose carboxymethyl ether sodium salt.

As indicated above, sustained and/or controlled release may be achieved by blending the L-arginine, derivative or salt with a polymer or a polymer matrix. Microspheres may be used for sustained and/or controlled release. Such microspheres may have differentially soluble coatings, for example, the same coating at different thicknesses or coatings soluble under different conditions, for example at different pH values as in the case of enteric coatings. If an enteric coating is also used, the active ingredient is released at the desired location and sustained or controlled release of the active ingredient in downstream regions is also achieved.

Drug delivery systems that are colon specific may also be used in order to effect release of the active substance in the colon. Drug delivery systems are known that effect selective targeting to the ascending, transverse or descending sections of the colon. For example, coatings are known that are degraded by colonic bacteria. Again, such a coating may be used in conjunction with a sustained and/or controlled release formulation.

Medicaments for use as a laxative are, for example, as described above for treatment of endogenous constipation.

The invention further provides the use of L-arginine, a derivative thereof, or a pharmaceutically acceptable salt thereof for the manufacture of a topical preparation for use as a laxative in a patient without constipation who should be prevented from straining at the stool, for example a patient who suffers from a hernia or cardiovascular disease, haemorrhoids or other anorectal disorders, for the treatment of endogenous constipation, or for clearing the rectum and/or the colon of a mammal, wherein the colon and/or rectum are cleared prior to carrying out a diagnostic, therapeutic or surgical procedure on the colon, rectum or anus or elsewhere in the abdomen, said preparation comprising:
- a therapeutically effective amount of L-arginine, a derivative thereof or a pharmaceutically acceptable salt thereof at a concentration of more than 100 mg L-arginine/ml;
- a pharmaceutically acceptable carrier, said carrier comprising a vehicle for topical administration of the L-arginine to an area of the gastrointestinal tract, said pharmaceutically acceptable carrier selected from a water soluble jelly, an anal suppository, an enema solution and a solution.

The present invention makes use of a medicament comprising L-arginine, a derivative thereof or a salt thereof, in admixture with a pharmaceutically acceptable carrier, the medicament comprising one or more of the following means: a coating that dissolves at a desired location in the - gastrointestinal tract; a sustained release formulation; a controlled release formulation and a drug delivery system. The means or combination of means are such that the L-arginine is released at the desired area of the gastrointestinal trace in a sustained and/or controlled manner.

The present invention makes use of a medicament comprising L-arginine, a derivative thereof or a sale thereof, in admixture with a pharmaceutically acceptable carrier, the medicament comprising an enteric coating that dissolves at a desired location in the gastrointestinal tract, especially in the terminal ileum.

The present invention makes use of a medicament comprising L-arginine, a derivative thereof or a salt thereof, in admixture with a pharmaceutically acceptable carrier, the medicament having a sustained or controlled release formulation.

The present invention makes use of a medicament comprising L-arginine, a derivative thereof or a salt thereof, in admixture with a pharmaceutically acceptable carrier comprising a colon-specific drug delivery system.

The amount of the L-arginine, derivative thereof or salt thereof to be administered may be from 0.001 mg, for example, from 0.1 mg, for example, from 0.5 mg, for example, from 4 mg, for example, from 10 mg, for example, from 50 mg, calculated as L-arginine. The amount administered may be up to 8000 mg, for example, up to 7000 mg, for example, up to 5000 mg, for example, up to 4000 mg for example, up to 1000 mg, for example up to 800 mg, calculated as L-arginine. Preferred ranges may be selected from any of the lower values set out above in combination with any of the upper values. Particularly preferred ranges for particular applications are given above.

A nutritional supplement comprising a Serona repens extract, at least one mineral, at least one vitamin and, optionally L-arginine is described in GB 2,323,531. Medicaments for use as laxatives according to the present invention preferably do not comprise the following combination of substances: Serona repens extract, at least one mineral, and at least one vitamin.

The following non-limiting Examples illustrate the invention.

### EXAMPLES:

1. Application of L-arginine to patients with anal fissures.
   28 patients suffering from anal fissures were recruited. Each patient was treated with a 4 ml dose of L-arginine gel. The L-arginine in water based jelly (here "K-Y Jelly" as supplied by Johnson and Johnson) was made up to contain 100 mg L-arginine per ml of gel. The L-arginine gel was applied into the anal canal with use of an applicator. In each patient, once the gel had diffused, rapid and uncontrollable defecation occurred. No other side effects, (e.g. headaches) were noted.
2. Application of L-arginine to the rectum in healthy volunteers.
   Six healthy volunteers, (3 male, 3 female, median age 46, range 23-51 years) were recruited. One of the subjects was known to have a history of migraine.
   Each patient was treated with a 1 ml dose of L-arginine gel, the gel having been made up to contain 400 mg of L-arginine per ml of hydroxymethyl cellulose. The 1 ml of L-arginine gel was applied from a 2.5 ml syringe directly into the rectum by ejection of 2 ml of gel from a 2.5 ml syringe with an attached canula with a known dead-volume of 1 ml. The canula was inserted 6 cm into the anus so that the gel was brought into contact with the rectum.
   All six of the subjects experienced a severe urge to defecate, which was accompanied by slimy diarrhoea in three of them. None of the subjects experienced any headaches.

## Claims

1. Use of L-arginine, or a derivative thereof, or a pharmaceutically acceptable salt thereof for the manufacture of a medicament for the treatment of endogenous constipation.

2. Use of L-arginine, a derivative thereof or a pharmaceutically acceptable salt thereof for the manufacture of a medicament for use as a laxative in a patient without constipation who should be prevented from straining at the stool, for example a patient who suffers from a hernia or cardiovascular disease, haemorrhoids or other anorectal disorders.

3. Use of L-arginine, a derivative thereof, or a pharmaceutically acceptable salt thereof for the manufacture of a medicament for clearing the rectum and/or the colon of a mammal, wherein the colon and/or rectum are cleared prior to carrying out a diagnostic, therapeutic or surgical procedure on the colon, rectum or anus or elsewhere in the abdomen.

4. Use as claimed in claim 3 wherein the diagnostic or surgical procedure is a colonoscopy or sigmoidoscopy,

5. Use as claimed in any one of claims 1 to 4 wherein the L-arginine or derivative thereof is L-arginine.

6. Use as claimed in any one of claims 1 to 5 wherein the pharmaceutically acceptable salt of L-arginine is L-arginine hydrochloride.

7. Use as claimed in any one of claims 1 to 6 wherein the medicament is suitable for topical application to an area of the gastrointestinal tract.

8. Use as claimed in any one of claims 1 to 7 wherein the medicament is suitable for rectal administration.

9. Use as claimed in claim 8 wherein the medicament comprises a pharmaceutically acceptable carrier which is a water-soluble jelly, a hydrogel or hydroxy methyl cellulose.

10. Use as claimed in claim 9 wherein the medicament comprises a pharmaceutically acceptable carrier which is an anal suppository base, or a cream.

11. use as claimed in claim 8 wherein the medicament comprises a pharmaceutically acceptable carrier that is an enema solution.

12. Use as claimed in any one of claims 1 to 11 in which the L-arginine, derivative thereof, or a pharmaceutically acceptable salt thereof has a concentration in the medicament of from 0.1 mg to 800 mg per ml, calculated as L-arginine.

13. Use as claimed in any one of claims 1 to 11 in which the L-arginine, derivative thereof, or a pharmaceutically acceptable salt thereof has a concentration in the medicament of from 10 mg to 700 mg per ml, calculated as L-arginine.

14. Use as claimed in any one of claims 1 to 11 in which the L-arginine, derivative thereof, or a pharmaceutically acceptable salt thereof has a concentration in the medicament of from 50 mg to 500 mg per ml, calculated as L-arginine.

15. Use as claimed in any one of claims 1 to 11 in which the L-arginine, derivative thereof, or a pharmaceutically acceptable salt thereof has a concentration in the medicament of 400 mg per ml, calculated as L-arginine.

16. Use as claimed in any one of claims 12 to 15, wherein from 0.1 to 800 mg of the medicament are administered.

17. Use as claimed in any one of claims 1 to 6 wherein the medicament is suitable for oral administration.

18. Use as claimed in claim 17 wherein the medicament is a tablet, a capsule or a powder.

19. Use as claimed in claim 17 wherein the medicament comprises one or more means selected from an enteric coating that dissolves at a desired location in the gastrointestinal tract; a controlled release formulation; a sustained release formulation; a targeted drug delivery system.

20. Use as claimed in claim 19 wherein the means are such that the L-arginine, derivative thereof, or pharmaceutically acceptable salt thereof is applied to a specific area of the gastrointestinal tract.

21. Use as claimed in claim 19 wherein the area of the gastrointestinal tract is the gastrointestinal tract beyond the ileum.

22. Use as claimed in claim 19 wherein the area of the gastrointestinal tract is the colon and beyond.

23. Use as claimed in claim 19 wherein the area of the gastrointestinal tract is the rectum or anal canal.

24. Use as claimed in any one of claims 1 to 23 wherein the medicament further comprises a bulk-forming laxative, an osmotic laxative, a stimulatory laxative or a softening agent for simultaneous, separate or sequential administration.

25. Use as claimed in any one of claims 1 to 24 wherein from 0.1 mg to 5 g of L-arginine reach the rectum, anus or colon.

26. Use as claimed in any one of claims 1 to 24, wherein from 10 mg to 1 g of L-arginine reach the rectum, anus or colon.

27. Use as claimed in any one of claims 1 to 24, wherein from 50 mg to 800 mg of L-arginine reach the rectum, anus or colon.

28. Use of L-arginine, a derivative thereof, or a pharmaceutically acceptable salt thereof for the manufacture of a topical preparation for use as a laxative in a patient without constipation who should be prevented from straining at the stool, for example a patient who suffers from a hernia or cardiovascular disease, haemorrhoids or other anorectal disorders, for the treatment of endogenous constipation, or for clearing the rectum and/or the colon of a mammal, wherein the colon and/or rectum are cleared prior to carrying out a diagnostic, therapeutic or surgical procedure on the colon, rectum or anus or elsewhere in the abdomen, said preparation comprising:
- a therapeutically effective amount of L-arginine, a derivative thereof or a pharmaceutically acceptable salt thereof at a concentration of more than 100 mg L-arginine/ml;
- a pharmaceutically acceptable carrier, said carrier comprising a vehicle for topical administration of the L-arginine to an area of the gastrointestinal tract, said pharmaceutically acceptable carrier selected from a water soluble jelly, an anal suppository, an enema solution and a solution.

29. L-arginine, a derivative thereof, or a pharmaceutically acceptable salt thereof for use in the treatment of endogenous constipation; for use as a laxative in a patient without constipation who should be prevented from straining at the stool, for example a patient who suffers from a hernia or cardiovascular disease, haemorrhoids or other anorectal disorders; or for use in clearing the rectum and/or the colon of a mammal, wherein the colon and/or rectum are cleared prior to carrying out a diagnostic, therapeutic or surgical procedure on the colon, rectum or anus or elsewhere in the abdomen.

30. L-arginine, a derivative thereof, or a pharmaceutically acceptable salt thereof as claimed in claim 29 wherein the pharmaceutically acceptable salt of L-arginine is L-arginine hydrochloride.

31. L-arginine, a derivative thereof, or a pharmaceutically acceptable salt thereof as claimed in claim 29 or 30 in which the L-arginine, derivative thereof, or a pharmaceutically acceptable salt thereof is in a medicament at a concentration of from 0.1 mg to 800 mg per ml, calculated as L-arginine.

32. L-arginine, a derivative thereof, or a pharmaceutically acceptable salt thereof as claimed in claim 29 or 30 in which the L-arginine, derivative thereof, or a pharmaceutically acceptable salt thereof is in a medicament at a concentration of 400 mg per ml, calculated as L-arginine.

33. L-arginine, a derivative thereof, or a pharmaceutically acceptable salt thereof for use as a laxative in a patient without constipation who should be prevented from straining at the stool, for example a patient who suffers from a hernia or cardiovascular disease, haemorrhoids or other anorectal disorders, for the treatment of endogenous constipation, or for clearing the rectum and/or the colon of a mammal, wherein the colon and/or rectum are cleared prior to carrying out a diagnostic, therapeutic or surgical procedure on the colon, rectum or anus or elsewhere in the abdomen, wherein said L-arginine a derivative thereof or a pharmaceutically acceptable salt thereof is in a preparation comprising:
- a therapeutically effective amount of L-arginine, a derivative thereof or a pharmaceutically acceptable salt thereof at a concentration of more than 100 mg L-arginine/ml;
- a pharmaceutically acceptable carrier, said carrier comprising a vehicle for topical administration of the L-arginine to an area of the gastrointestinal tract, said pharmaceutically acceptable carrier selected from a water soluble jelly, an anal suppository, an enema solution and a solution.

## Patentansprüche

1. Verwendung von L-Arginin oder eines Derivats davon oder eines pharmazeutisch annehmbaren Salzes davon zur Herstellung eines Medikaments zur Behandlung von endogener Obstipation.

2. Verwendung von L-Arginin, eines Derivats davon oder eines pharmazeutisch annehmbaren Salzes davon zur Herstellung eines Medikaments zur Verwendung als ein Abführmittel bei einem Patienten ohne Obstipation, der beim Stuhlgang Pressen vermeiden soll, zum Beispiel bei einem Patienten, der an Leistenbruch oder Herz-Kreislauf-Erkrankung, Hämorriden oder anderen anorektalen Störungen leidet.

3. Verwendung von L-Arginin, eines Derivats davon oder eines pharmazeutisch annehmbaren Salzes davon zur Herstellung eines Medikaments zur Leerung des Rektums und/oder des Kolons eines Säugers, wobei das Kolon und/oder Rektum vor der Durchführung eines diagnostischen, therapeutischen oder operativen Verfahrens am Kolon, Rektum oder Anus oder anderswo im Abdomen geleert werden/wird.

4. Verwendung wie in Anspruch 3 beansprucht, wobei das diagnostische oder operative Verfahren eine Kolonoskopie oder Sigmoidoskopie ist.

5. Verwendung wie in irgendeinem der Ansprüche 1 bis 4 beansprucht, wobei das L-Arginin oder Derivat davon L-Arginin ist.

6. Verwendung wie in irgendeinem der Ansprüche 1 bis 5 beansprucht, wobei das pharmazeutisch annehmbare Salz von L-Arginin L-Arginin-Hydrochlorid ist.

7. Verwendung wie in irgendeinem der Ansprüche 1 bis 6 beansprucht, wobei das Medikament zum topischen Auftrag auf einen Bereich des Gastrointestinaltraktes geeignet ist.

8. Verwendung wie in irgendeinem der Ansprüche 1 bis 7 beansprucht, wobei das Medikament zur rektalen Verabreichung geeignet ist.

9. Verwendung wie in Anspruch 8 beansprucht, wobei das Medikament einen pharmazeutisch annehmbaren Träger enthält, der ein wasserlösliches Gel, ein Hydrogel oder Hydroxymethylcellulose ist.

10. Verwendung wie in Anspruch 8 beansprucht, wobei das Medikament einen pharmazeutisch annehmbaren Träger enthält, der ein Analzäpfchengrundstoff oder eine Creme ist.

11. Verwendung wie in Anspruch 8 beansprucht, wobei das Medikament einen pharmazeutisch annehmbaren Träger enthält, der eine Darmspüllösung ist.

12. Verwendung wie in irgendeinem der Ansprüche 1 bis 11 beansprucht, wobei das L-Arginin, ein Derivat davon oder ein pharmazeutisch annehmbares Salz davon eine Konzentration in dem Medikament von 0,1 mg bis 800 mg pro ml, berechnet als L-Arginin, besitzt.

13. Verwendung wie in irgendeinem der Ansprüche 1 bis 11 beansprucht, wobei das L-Arginin, ein Derivat davon oder ein pharmazeutisch annehmbares Salz davon eine Konzentration in dem Medikament von 10 mg bis 700 mg pro ml, berechnet als L-Arginin, besitzt.

14. Verwendung wie in irgendeinem der Ansprüche 1 bis 11 beansprucht, wobei das L-Arginin, ein Derivat davon oder ein pharmazeutisch annehmbares Salz davon eine Konzentration in dem Medikament von 50 mg bis 500 mg pro ml, berechnet als L-Arginin, besitzt.

15. Verwendung wie in irgendeinem der Ansprüche 1 bis 11 beansprucht, wobei das L-Arginin, ein Derivat davon oder ein pharmazeutisch annehmbares Salz davon eine Konzentration in dem Medikament von 400 mg pro ml, berechnet als L-Arginin, besitzt.

16. Verwendung wie in irgendeinem der Ansprüche 12 bis 15 beansprucht, wobei 0,1 bis 800 mg des Medikaments verabreicht werden.

17. Verwendung wie in irgendeinem der Ansprüche 1 bis 6 beansprucht, wobei das Medikament zur oralen Verabreichung geeignet ist.

18. Verwendung wie in Anspruch 17 beansprucht, wobei das Medikament eine Tablette, eine Kapsel oder ein Pulver ist.

19. Verwendung wie in Anspruch 17 beansprucht, wobei das Medikament eines oder mehrere Mittel enthält, ausgewählt aus einer magensaftresistenten Beschichtung, die sich am erwünschten Ort im Gastrointestinaltrakt auflöst, einer Formulierung mit gesteuerter Freisetzung, einer Formulierung mit verzögerter Freisetzung, einem zielgeführten Arzneimittelabgabesystem.

20. Verwendung wie in Anspruch 19 beansprucht, wobei die Mittel derart sind, dass das L-Arginin, ein Derivat davon oder ein pharmazeutisch annehmbares Salz davon auf einen speziellen Bereich des Gastrointestinaltraktes aufgetragen wird.

21. Verwendung wie in Anspruch 19 beansprucht, wobei der Bereich des Gastrointestinaltraktes der Gastrointestinaltrakt jenseits des Ileums ist.

22. Verwendung wie in Anspruch 19 beansprucht, wobei der Bereich des Gastrointestinaltraktes das Kolon und darüber hinaus ist.

23. Verwendung wie in Anspruch 19 beansprucht, wobei der Bereich des Gastrointestinaltraktes das Rektum oder der Analkanal ist.

24. Verwendung wie in irgendeinem der Ansprüche 1 bis 23 beansprucht, wobei das Medikament ferner ein quellendes Abführmittel, ein osmotisches Abführmittel, ein stimulierendes Abführmittel oder ein erweichendes Mittel zur gleichzeitigen, separaten oder sequentiellen Verabreichung enthält.

25. Verwendung wie in irgendeinem der Ansprüche 1 bis 24 beansprucht, wobei 0,1 mg bis 5 g L-Arginin das Rektum, den Anus oder das Kolon erreichen.

26. Verwendung wie in irgendeinem der Ansprüche 1 bis 24 beansprucht, wobei 10 mg bis 1 g L-Arginin das Rektum, den Anus oder das Kolon erreichen.

27. Verwendung wie in irgendeinem der Ansprüche 1 bis 24 beansprucht, wobei 50 mg bis 800 mg L-Arginin das Rektum, den Anus oder das Kolon erreichen.

28. Verwendung von L-Arginin, eines Derivats davon oder eines pharmazeutisch annehmbaren Salzes davon zur Herstellung eines topischen Präparats zur Verwendung als ein Abführmittel bei einem Patienten ohne Obstipation, der beim Stuhlgang Pressen vermeiden soll, zum Beispiel bei einem Patienten, der an Leistenbruch oder Herz-Kreislauf-Erkrankung, Hämorriden oder anderen anorektalen Störungen leidet, zur Behandlung von endogener Obstipation oder zur Leerung des Rektums und/oder des Kolons eines Säugers, wobei das Kolon und/oder Rektum vor der Durchführung eines diagnostischen, therapeutischen oder operativen Verfahrens am Kolon, Rektum oder Anus oder anderswo im Abdomen geleert werden/wird, wobei das Präparat enthält:
- eine therapeutisch wirksame Menge L-Arginin, eines Derivats davon oder eines pharmazeutisch annehmbaren Salzes davon in einer Konzentration von mehr als 100 mg L-Arginin/ml,
- einen pharmazeutisch annehmbaren Träger, wobei der Träger ein Vehikel zur topischen Verabreichung des L-Arginins an einen Bereich des Gastrointestinaltraktes enthält, wobei der pharmazeutisch annehmbare Träger ausgewählt ist aus wasserlöslichem Gel, einem Analzäpfchen, einer Darmspüllösung und einer Lösung.

29. L-Arginin, ein Derivat davon oder ein pharmazeutisch annehmbares Salz davon zur Verwendung bei der Behandlung von endogener Obstipation, zur Verwendung als ein Abführmittel bei einem Patienten ohne Obstipation, der beim Stuhlgang Pressen vermeiden soll, zum Beispiel bei einem Patienten, der an Leistenbruch oder Herz-Kreislauf-Erkrankung, Hämorriden oder anderen anorektalen Störungen leidet, oder zur Verwendung bei der Leerung des Rektums und/oder des Kolons eines Säugers, wobei das Kolon und/oder Rektum vor der Durchführung eines diagnostischen, therapeutischen oder operativen Verfahrens am Kolon, Rektum oder Anus oder anderswo im Abdomen geleert werden/wird.

30. L-Arginin, ein Derivat davon oder ein pharmazeutisch annehmbares Salz davon wie in Anspruch 29 beansprucht, wobei das pharmazeutisch annehmbare Salz von L-Arginin L-Arginin-Hydrochlorid ist.

31. L-Arginin, ein Derivat davon oder ein pharmazeutisch annehmbares Salz davon wie in Anspruch 29 oder 30 beansprucht, wobei das L-Arginin, ein Derivat davon oder ein pharmazeutisch annehmbares Salz davon in einem Medikament in einer Konzentration von 0,1 mg bis 800 mg pro ml, berechnet als L-Arginin, vorliegt.

32. L-Arginin, ein Derivat davon oder ein pharmazeutisch annehmbares Salz davon wie in Anspruch 29 oder 30 beansprucht, wobei das L-Arginin, ein Derivat davon oder ein pharmazeutisch annehmbares Salz davon in einem Medikament in einer Konzentration von 400 mg pro ml, berechnet als L-Arginin, vorliegt.

33. L-Arginin, ein Derivat davon oder ein pharmazeutisch annehmbares Salz davon zur Verwendung als ein Abführmittel bei einem Patienten ohne Obstipation, der beim Stuhlgang Pressen vermeiden soll, zum Beispiel bei einem Patienten, der an Leistenbruch oder Herz-Kreislauf-Erkrankung, Hämorriden oder anderen anorektalen Störungen leidet, zur Behandlung von endogener Obstipation oder zur Leerung des Rektums und/oder des Kolons eines Säugers, wobei das Kolon und/oder Rektum vor der Durchführung eines diagnostischen, therapeutischen oder operativen Verfahrens am Kolon, Rektum oder Anus oder anderswo im Abdomen geleert werden/wird, wobei das L-Arginin, ein Derivat davon oder ein pharmazeutisch annehmbares Salz davon in einem Präparat vorliegt, das enthält:
- eine therapeutisch wirksame Menge L-Arginin, eines Derivats davon oder eines pharmazeutisch annehmbaren Salzes davon in einer Konzentration von mehr als 100 mg L-Arginin/ml,
- einen pharmazeutisch annehmbaren Träger, wobei der Träger ein Vehikel zur topischen Verabreichung des L-Arginins an einen Bereich des Gastrointestinaltraktes enthält, wobei der pharmazeutisch annehmbare Träger ausgewählt ist aus wasserlöslichem Gel, einem Analzäpfchen, einer Darmspüllösung und einer Lösung.

## Revendications

1. Utilisation de la L-arginine, d'un dérivé de celle-ci ou d'un de ses sels acceptables sur le plan pharmaceutique pour la fabrication d'un médicament destiné au traitement de la constipation endogène.

2. Utilisation de la L-arginine, d'un dérivé de celle-ci ou d'un de ses sels acceptables sur le plan pharmaceutique pour la fabrication d'un médicament destiné à une utilisation en tant que laxatif chez un patient non constipé qui ne doit pas forcer à la selle, par exemple un patient souffrant d'une hernie ou d'une maladie cardiovasculaire, d'hémorroïdes ou d'autres troubles ano-rectaux.

3. Utilisation de la L-arginine, d'un dérivé de celle-ci ou d'un de ses sels acceptables sur le plan pharmaceutique pour la fabrication d'un médicament destiné au lavement du rectum et/ou du côlon d'un mammifère, dans laquelle le côlon et/ou le rectum sont lavés avant la réalisation d'une intervention diagnostique, thérapeutique ou chirurgicale sur le côlon, le rectum ou l'anus ou dans une autre partie de l'abdomen.

4. Utilisation selon la revendication 3, dans laquelle l'intervention diagnostique ou chirurgicale est une coloscopie ou une sigmoïdoscopie.

5. Utilisation selon l'une quelconque des revendications 1 à 4, dans laquelle la L-arginine ou le dérivé de celle-ci est la L-arginine.

6. Utilisation selon l'une quelconque des revendications 1 à 5, dans laquelle le sel acceptable sur le plan pharmaceutique de la L-arginine est le chlorhydrate de L-arginine.

7. Utilisation selon l'une quelconque des revendications 1 à 6, dans laquelle le médicament est approprié pour une application topique sur une zone du tractus gastro-intestinal.

8. Utilisation selon l'une quelconque des revendications 1 à 7, dans laquelle le médicament est approprié pour une administration rectale.

9. Utilisation selon la revendication 8, dans laquelle le médicament comprend un support acceptable sur le plan pharmaceutique qui est une gelée hydrosoluble, un hydrogel ou l'hydroxyméthylcellulose.

10. Utilisation selon la revendication 8, dans laquelle le médicament comprend un support acceptable sur le plan pharmaceutique qui est une base de suppositoire ou une crème.

11. Utilisation selon la revendication 8, dans laquelle le médicament comprend un support acceptable sur le plan pharmaceutique qui est une solution pour lavement.

12. Utilisation selon l'une quelconque des revendications 1 à 11, dans laquelle la L-arginine, le dérivé de celle-ci ou un de ses sels acceptables sur le plan pharmaceutique a une concentration dans le médicament allant de 0,1 mg à 800 mg par mL, exprimée en L-arginine.

13. Utilisation selon l'une quelconque des revendications 1 à 11, dans laquelle la L-arginine, le dérivé de celle-ci ou un de ses sels acceptables sur le plan pharmaceutique a une concentration dans le médicament allant de 10 mg à 700 mg par mL, exprimée en L-arginine.

14. Utilisation selon l'une quelconque des revendications 1 à 11, dans laquelle la L-arginine, le dérivé de celle-ci ou un de ses sels acceptables sur le plan pharmaceutique a une concentration dans le médicament allant de 50 mg à 500 mg par mL, exprimée en L-arginine.

15. Utilisation selon l'une quelconque des revendications 1 à 11, dans laquelle la L-arginine, le dérivé de celle-ci ou un de ses sels acceptables sur le plan pharmaceutique a une concentration dans le médicament de 400 mg par mL, exprimée en L-arginine.

16. Utilisation selon l'une quelconque des revendications 12 à 15, dans laquelle de 0,1 à 800 mg du médicament sont administrés.

17. Utilisation selon l'une quelconque des revendications 1 à 6, dans laquelle le médicament est approprié pour une administration orale.

18. Utilisation selon la revendication 17, dans laquelle le médicament est un comprimé, une gélule ou une poudre.

19. Utilisation selon la revendication 17, dans laquelle le médicament comprend un ou plusieurs moyens choisis parmi un enrobage gastro-résistant qui se dissout à un endroit souhaité dans le tractus gastro-intestinal ; une formulation à libération modifiée ; une formulation à libération prolongée ; un système de délivrance de médicament ciblé.

20. Utilisation selon la revendication 19, dans laquelle les moyens sont tels que la L-arginine, le dérivé de celle-ci, ou son sel acceptable sur le plan pharmaceutique est appliqué sur une zone spécifique du tractus gastro-intestinal.

21. Utilisation selon la revendication 19, dans laquelle la zone du tractus gastro-intestinal est le tractus gastro-intestinal situé au-delà de l'iléon.

22. Utilisation selon la revendication 19, dans laquelle la zone du tractus gastro-intestinal est le côlon et la partie au-delà.

23. Utilisation selon la revendication 19, dans laquelle la zone du tractus gastro-intestinal est le rectum ou le canal anal.

24. Utilisation selon l'une quelconque des revendications 1 à 23, dans laquelle le médicament comprend en outre un laxatif de lest, un laxatif osmotique, un laxatif stimulant ou un laxatif émollient destinés à une administration simultanée, séparée ou séquentielle.

25. Utilisation selon l'une quelconque des revendications 1 à 24, dans laquelle de 0,1 mg à 5 g de L-arginine atteignent le rectum, l'anus ou le côlon.

26. Utilisation selon l'une quelconque des revendications 1 à 24, dans laquelle de 10 mg à 1 g de L-arginine atteignent le rectum, l'anus ou le côlon.

27. Utilisation selon l'une quelconque des revendications 1 à 24, dans laquelle de 50 mg à 800 mg de L-arginine atteignent le rectum, l'anus ou le côlon.

28. Utilisation de la L-arginine, d'un dérivé de celle-ci ou d'un de ses sels acceptables sur le plan pharmaceutique pour la fabrication d'une préparation topique destinée à une utilisation en tant que laxatif chez un patient non constipé qui ne doit pas forcer à la selle, par exemple un patient souffrant d'une hernie ou d'une maladie cardiovasculaire, d'hémorroïdes ou d'autres troubles ano-rectaux, pour le traitement de la constipation endogène ou pour le lavement du rectum et/ou du côlon d'un mammifère, dans laquelle le côlon et/ou le rectum sont lavés avant la réalisation d'une intervention diagnostique, thérapeutique ou chirurgicale sur le côlon, le rectum ou l'anus ou dans une autre partie de l'abdomen, ladite préparation comprenant :
- une quantité efficace sur le plan thérapeutique de L-arginine, d'un dérivé de celle-ci ou d'un de ses sels acceptables sur le plan pharmaceutique à une concentration de plus de 100 mg de L-arginine/mL ;
- un support acceptable sur le plan pharmaceutique, ledit support comprenant un véhicule permettant une administration topique de L-arginine sur une zone du tractus gastro-intestinal, ledit support acceptable sur le plan pharmaceutique étant choisi parmi une gelée hydrosoluble, un suppositoire, une solution pour lavement et une solution.

29. L-arginine, dérivé de celle-ci ou un de ses sels acceptables sur le plan pharmaceutique pour une utilisation dans le traitement de la constipation endogène ; pour une utilisation en tant que laxatif chez un patient non constipé qui ne doit pas forcer à la selle, par exemple un patient souffrant d'une hernie ou d'une maladie cardiovasculaire, d'hémorroïdes ou d'autres troubles ano-rectaux ; ou pour une utilisation dans le lavement du rectum et/ou du côlon d'un mammifère, dans lequel le côlon et/ou le rectum sont lavés avant la réalisation d'une intervention diagnostique, thérapeutique ou chirurgicale sur le côlon, le rectum ou l'anus ou dans une autre partie de l'abdomen.

30. L-arginine, dérivé de celle-ci ou un de ses sels acceptables sur le plan pharmaceutique selon la revendication 29, dans lequel le sel acceptable sur le plan pharmaceutique de L-arginine est le chlorhydrate de L-arginine.

31. L-arginine, dérivé de celle-ci ou un de ses sels acceptables sur le plan pharmaceutique selon la revendication 29 ou 30, dans lequel la L-arginine, le dérivé de celle-ci ou un de ses sels acceptables sur le plan pharmaceutique est présent dans un médicament à une concentration allant de 0,1 mg à 800 mg par mL, exprimée en L-arginine.

32. L-arginine, dérivé de celle-ci ou un de ses sels acceptables sur le plan pharmaceutique selon la revendication 29 ou 30, dans lequel la L-arginine, le dérivé de celle-ci ou un de ses sels acceptables sur le plan pharmaceutique est présent dans un médicament à une concentration de 400 mg par mL, exprimée en L-arginine.

33. L-arginine, dérivé de celle-ci ou un de ses sels acceptables sur le plan pharmaceutique pour une utilisation en tant que laxatif chez un patient non constipé qui ne doit pas forcer à la selle, par exemple un patient souffrant d'une hernie ou d'une maladie cardiovasculaire, d'hémorroïdes ou d'autres troubles ano-rectaux, pour le traitement de la constipation endogène ou pour le lavement du rectum et/ou du côlon d'un mammifère, dans lequel le côlon et/ou le rectum sont lavés avant la réalisation d'une intervention diagnostique, thérapeutique ou chirurgicale sur le côlon, le rectum ou l'anus ou dans une autre partie de l'abdomen, dans lequel ladite L-arginine, dérivé de celle-ci ou un de ses sels acceptables sur le plan pharmaceutique est présent dans une préparation comprenant :
- une quantité efficace sur le plan thérapeutique de L-arginine, d'un dérivé de celle-ci ou d'un de ses sels acceptables sur le plan pharmaceutique à une concentration de plus de 100 mg de L-arginine/mL ;
- un support acceptable sur le plan pharmaceutique, ledit support comprenant un véhicule permettant une administration topique de L-arginine sur une zone du tractus gastro-intestinal, ledit support acceptable sur le plan pharmaceutique étant choisi parmi une gelée hydrosoluble, un suppositoire, une solution pour lavement et une solution.
